# EUROPEAN PATENT APPLICATION

(11) **EP 3 460 800 A1**
(43) Date of publication of application: **27.03.2019**
(21) Application number: 17192139.8
(22) Date of filing: 20.09.2017
(51) Int. Cl.: G16H 10/00, G16H 20/00, G16H 40/00

(54) **PROVIDING ORDERED CLINICAL INFORMATION**

(71) Applicant: Koninklijke Philips N.V., 5656 AE Eindhoven (NL)
(72) Inventor: SERLIE, Iwo Willem Oscar, 5656 AE Eindhoven (NL)
(74) Representative: de Haan, Poul Erik

(57) **Abstract**

Presented are concepts for providing ordered clinical information. One such concept comprises obtaining a clinical map, clinical data comprising a plurality of clinical data elements and display preference information representative of a preference for exploring clinical data. The clinical data is then processed based on the clinical map and the obtained display preference information to generate an ordered set of clinical data elements.

## Description

### FIELD OF THE INVENTION

This invention relates to the field of clinical information provision and more particularly to the provision of ordered clinical information.

### BACKGROUND OF THE INVENTION

Known systems for providing clinical information (such as subject data) are typically arranged to provide (e.g. display) a list of clinical data elements, such as a list of values for clinical parameters.

A conventional approach to providing such a list includes ordering the data elements alphabetically. When the clinical data elements are ordered alphabetically, a user (e.g. doctor, clinician, technician, medical professional, etc.) may be provided with a list of items from which it may be required to find a desired or relevant data element. To address this issue, it has been proposed to provide a list of clinical data elements wherein the data elements are ordered by frequency of use. However, ordering the clinical data elements according to frequency of use may have the drawback that the ordering is irrelevant of a particular context concerned.

For a subject having numerous medical events (e.g. a subject suffering from chronic diseases and/or regularly visiting a medical practitioner), clinical information relating to the subject may be extensive. Exploration and/or analysis of the subject's clinical information may therefore be resource intensive and/or difficult. With intuitive presentation and navigation of subject-specific clinical information being of paramount importance in Clinical Decision Support (CDS), there remains a need for an approach to providing clinical information in an ordered manner which is simple, intuitive and easy to navigate.

### SUMMARY OF THE INVENTION

The invention aims to at least partly fulfil the aforementioned needs. To this end, the invention provides devices, systems and methods as defined in the independent claims. The dependent claims provide advantageous embodiments.

There is provided a method for providing ordered clinical information. The method comprises: obtaining a clinical map, the clinical map comprising graphical information for graphically representing clinical data; obtaining clinical data comprising a plurality of clinical data elements; obtaining display preference information representative of a preference for exploring clinical data; and processing the clinical data to generate an ordered set of clinical data elements based on the clinical map and the obtained display preference information.

Proposed is a concept for generating an ordered set of clinical data (such as an ordered list of clinical values, or ordered 'value set'), wherein the clinical data is ordered according to a preference for exploring data in a graphical representation of clinical data (as represented or defined by a clinical map). Embodiments may thus propose the ordering of clinical data according to clinical patterns of education of a specific user and according to graphical representations of clinical objects in a clinical map.

Ordering of clinical data with respect to an exploration preference in a graphical representation of clinical data (as represented or defined by a clinical map for example) may facilitate the provision and/or display of clinical information that can be combined with the graphical representation for simplified exploration and/or analysis. When working with such clinical information and the graphical representation, clinical users may require less mental effort in switching between representations.

Combination of clinical data and a clinical map, wherein the clinical data is ordered in consideration of the clinical map and an exploration preference, may help a user to explore and/or analyse the clinical data in a simple and efficient manner. For example, a user's preferred direction, hierarchical ordering or trajectory for data exploration may be accounted for by sorting clinical data elements according to their projected distance along the preferred direction or trajectory.

Proposed concepts may thus increase the clinical value of information by enabling clinical information to be provided (e.g. displayed or graphically represented) in an ordered manner which accounts for an exploration preference (which may be defined by a pattern of clinical education for example). This may support assessment, enable improved focus on a diagnosis, and/or support clinical planning. Improved CDS may therefore be facilitated by proposed concepts. Also, proposed embodiments may support visual navigation through subject-specific clinical (e.g. diagnostic) information with respect to graphical representation of clinical data (as represented or defined by a clinical map for example). For instance, an embodiment may help a user to localize or regionalize a solution if different patterns of clinical education are employed.

Proposed embodiments may provide a set of ordered clinical information comprising structured health domain content that is specific to a subject. The provided clinical information may be structured in a hierarchy, thus expressing refinements of clinical information according to clinical semantics (e.g. a heart valve data may be a refinement of heart data). Such ordered clinical information may be generated with reference to an exploration direction in a clinical map, wherein a clinical map may be thought of a graphical interaction paradigm with structured clinical data. For instance, a clinical map may comprise a graphical representation of clinical data, or a definition thereof

Typically, a clinical map comprises graphical information for graphically representing clinical data, wherein the clinical data may relate to a subject and/or to non-subject information (such as standards for interpretation or guidelines, for example). A clinical map may therefore provide a graphical representation of a region of anatomy, physiology and/or pathology of a human or animal. For instance, a clinical map may provide information for graphically representing clinical data relating to a heart, but may also be structured in a hierarchical form so that a refinement (e.g. sub-level) of the clinical model of the heart comprises a clinical model of a heart valve of the heart (e.g. information for graphically representing clinical data relating to the heart valve). A clinical map may thus comprise regions that are mapped to clinical data elements.

Accordingly, concepts are proposed which may enrich the display or visualisation of clinical information relating to a specific subject by creating a subject-specific ordered clinical information using a clinical map, wherein the ordering of the clinical information is based on a preference of exploration in the clinical map. The ordered information and the clinical map(s) may be combined and thus facilitate simple visualisation and navigation of clinical information.

Some proposed embodiments may be thought of as ordering clinical information (such as a set of clinical values) according to clinical patterns of education of a specific user, user group, department, region, etc. and according to graphical representations of clinical objects in a clinical map. A user may therefore be provided with clinical information having an ordering which is more relevant to requirements/needs.

In an embodiment, the clinical data may comprise a list of a plurality of clinical data elements. The step of processing the clinical data may then comprise: ordering the plurality of clinical data elements according to the preference for exploring clinical data so as to generate an ordered list of clinical data elements; and sorting clinical data elements of the ordered list based on the clinical map so as to generate a sorted ordered list of clinical data elements.

The clinical map may be representative of an anatomical relationship between anatomical features of the subject. This may support the presentation and/or navigation of subject-specific clinical information in a manner which is representative a relative location and/or relationship in a subject's body. Simple and intuitive presentation or navigation of clinical information may thus be facilitated by proposed embodiments. For instance, by positioning clinical maps in a graphical representation of a subject's body according to the location of the associated anatomical feature(s), a clinical model may be provided which enables quick and easy navigation of the clinical maps.

Each clinical data element may comprise level information describing a hierarchical level it relates to. The step of processing the clinical data may then comprise ordering the plurality of clinical data elements in a hierarchical manner based on the level information of the clinical data elements.

In an embodiment, the clinical map may comprise hierarchy information representative of a hierarchy of anatomical features of a human or animal body. The step of processing the clinical data may then comprise ordering the plurality of clinical data elements in a hierarchical manner based on the hierarchy information of the clinical map.

Obtaining display preference information representative of a preference for exploring clinical data may comprise receiving a user input signal via a user interface, wherein the user input signal comprises display preference information indicating a user's preferred direction or trajectory for exploring clinical data. For example, there may be provided a user input interface for enabling a user to indicate a preference of the ordering, and the clinical data may then be ordered and provided (e.g. displayed) according to the preference that is input by the user.

For example, in some embodiments, the display preference information may comprise an indication of direction or trajectory in a graphical representation of the clinical data defined by the clinical map. For instance, one or more graphical representations may overlay a clinical map in a graphical representation of generated model. A preference of the user may therefore be input graphically, for example by manipulating an orientation of a graphical arrow that overlays graphical elements displayed in a clinical map representation. Orientation of the arrow may therefore be used to determine an order in which the clinical data elements are provided. Additionally, the user may indicate further preferences as stored in a database. Embodiments may therefore facilitate quick, easy and intuitive navigation of the clinical model.

Proposed embodiments may enable a user to quickly and intuitively obtain ordered clinical information which may, for example, then be displayed to the user when creating a structured report. By selecting a relevant anatomical area and an exploration order that the user prefers anatomical features to be provided in, the user need not constantly think about the names of the relevant anatomical features when switching between different anatomical regions. Also, embodiments may help cater for situations where the clinical information uses labels that are different or not exactly the same as what a user is searching for. By using an input interface, relevant clinical information may be automatically provided, thereby avoiding time scrolling down long lists of clinical information and/or the mental effort in having to remember the names of the various types of clinical information that might be relevant. Embodiments may therefore facilitate the ordering of clinical information in a manner which is tailored to a user's preference in relation to a clinical map. This may, in turn, facilitate the provision of clinical information that is ordered in a way that is more intuitive and/or convenient for the use to explore.

According to another aspect of the invention, there is provided a method for displaying clinical information. The method comprises: providing ordered clinical information according to a proposed embodiment; generating a display control signal for displaying clinical information based on ordered clinical information; and displaying clinical information in accordance with the generated display control signal.

Proposed embodiments may thus help a user (such as a medical practitioner, doctor, surgeon, technician, etc.) to be presented with clinical information that is ordered in manner which caters for individual user preference and/or a clinical context. Information ordered in consideration of a preferred direction, hierarchical structure or trajectory for exploration may enable a user to focus on particular clinical information of interest, thus reducing complexity or confusion that may otherwise be caused by presenting additional and/or irrelevant information.

Embodiments may increase a value of clinical information by using user and/or clinical criteria to provide the clinical information in an order which is optimised for a user.

The step of generating a display control signal may be further based on a display position of the clinical information. This can help to ensure that the ordered information is displayed in an optimal manner, for example by accounting for available display space, display rendering constraints, etc.

According to another aspect, there is provided a computer program product for providing or displaying clinical information, wherein the computer program product comprises a computer-readable storage medium having computer-readable program code embodied therewith, the computer-readable program code configured to perform all of the steps of a proposed embodiment.

There is also provided a computer system comprising: a computer program product according to proposed embodiment; and one or more processors adapted to perform a method according to a propose concept by execution of the computer-readable program code of said computer program product.

According to another aspect of the invention, there is provided a system for providing ordered clinical information. The system comprises: an input interface adapted to obtain a clinical map, the clinical map comprising graphical information for graphically representing clinical data, to obtain clinical data comprising a plurality of clinical data elements, and to obtain display preference information representative of a preference for exploring clinical data; and a processing module adapted to process the clinical data to generate an ordered set of clinical data elements based on the clinical map and the obtained display preference information.

In some embodiments, the clinical data may comprise a list of a plurality of clinical data elements. The processing module may then be adapted to: order the plurality of clinical data elements according to the preference for exploring clinical data so as to generate an ordered list of clinical data elements; and sort clinical data elements of the ordered list based on the clinical map so as to generate a sorted ordered list of clinical data elements.

According to yet another aspect of the invention, there is provided a system for displaying clinical information. The system comprises: a system for providing ordered clinical information according to a proposed embodiment; an output interface adapted to generate a display control signal for displaying clinical information based on ordered clinical information; and a display unit adapted to display clinical information in accordance with the generated display control signal.

The processing module may be remotely located from the display unit, and the display control signal may thus be communicated to the display unit via a communication link. In this way, a user (such as a medical professional) may have an appropriately arranged display system that can receive and display clinical information at a location remotely located from the processing module. Embodiments may therefore enable a user to remotely review ordered clinical information using a portable display device, such as a laptop, tablet computer, mobile phone, PDA, etc.

The system may further comprise: a server device comprising the data processing module; and a client device comprising the display unit. Dedicated data processing means may therefore be employed for the purpose of generating an ordered clinical information, and generating a display control signal, thus reducing processing requirements or capabilities of other components or devices of the system.

The system may further comprise a client device, wherein the client device comprises the data processing module and the display unit. In other words, a user (such as a doctor or medical professional) may have an appropriately arranged client device (such as a laptop, tablet computer, mobile phone, PDA, etc.) which processes received data in order to generate an ordered clinical information and generate a control signal.

Thus, it will be understood that processing capabilities may therefore be distributed throughout the system in different ways according to predetermined constraints and/or availability of processing resources.

These and other aspects of the invention will be apparent from and elucidated with reference to the embodiment(s) described hereinafter.

### BRIEF DESCRIPTION OF THE DRAWINGS

Examples in accordance with aspects of the invention will now be described in detail with reference to the accompanying schematic drawings, in which:
Figure 1 is an exemplary flow diagram of a method for providing ordered clinical information according to an embodiment;
Figure 2 is an exemplary flow diagram of a method for displaying ordered clinical information according to an embodiment;
Figure 3 is a simplified block diagram of a system for providing ordered clinical information according to an embodiment;
Figure 4 is a simplified block diagram of a system according to an embodiment; and
Figure 5 is a simplified block diagram of a computer within which one or more parts of an embodiment may be employed.

### DETAILED DESCRIPTION OF THE EMBODIMENTS

Proposed is a concept for enabling the provision of clinical information that is ordered in manner which takes account of information relating to a preference for exploring data in a graphical representation of clinical data (as represented or defined by a clinical map). Ordering of clinical information with respect to a preference (such as direction, hierarchical order or trajectory for example) in a graphical representation of clinical data (as represented or defined by a clinical map) may facilitate the provision and/or display of clinical information that can help a user to explore and/or analyse the clinical data in a simple and efficient manner.

Accordingly, concepts are proposed which may enrich the display or visualisation of clinical information relating to a specific subject by creating a subject-specific ordered clinical information using a clinical map, wherein the ordering of the clinical information is based on a preference for exploration in the clinical map.

For example, by positioning clinical maps in a graphical representation of a subject's body according to the location of the associated anatomical feature(s), a clinical model may be provided which enables quick and easy navigation of the clinical maps. Display preference information representative of a preference for exploring clinical data may then be obtained by receiving a user input signal via a user interface, wherein the user input signal indicates a user's preference for exploring clinical data. The clinical data may then be ordered and provided (e.g. displayed) according to the preference that is input by the user. For instance, in some embodiments, one or more graphical representations may overlay a clinical map in a graphical representation of generated model and a preference of the user may be input graphically (for example by manipulating an orientation of a graphical arrow that overlays graphical elements displayed in a clinical map representation). Orientation of a displayed graphical element (which can be manipulated by the user) may thus be used determine an order in which the clinical data elements are provided. Embodiments may therefore facilitate quick, easy and intuitive navigation of the clinical model by ordering the clinical information in consideration of a user-indicated preference.

Clinical maps may thus be combined with a subject-specific clinical information in a manner which takes account of user's preference for exploration. Clinical information provided by such proposals may thus facilitate simple and intuitive navigation and assessment of subject-specific clinical information with reference to a user-indicated exploration preference, for example by ordering clinical information based on a user-input specifying the exploration preference. Improved CDS may therefore be provided by proposed embodiments.

Embodiments of the present invention are therefore directed toward enabling the provision of clinical information with reference to user-preference and a clinical map so as to facilitate or enhance a CDS process. Further, embodiments may be aimed at enabling the display or visualisation of clinical information with simplicity, accuracy and/or clinical context while providing enough detail so that a clinical assessment or decision is facilitated or supported (preferably with increased efficiency and/or reliability).

Embodiments are based on the insight that the provision of clinical information may be enhanced by ordering the information in consideration of a preference for exploration in a clinical map.

A clinical (image) map may be a graphical representation of clinical data, and it is typically combined with a description of areas on the image (e.g. in an 'overlay' or multiple overlays). The areas are then linked to elements described in a clinical model.

A clinical model can thus be thought of as a formal representation of entities in a domain and their relations (e.g. abstractions that describe aspects of a muscular system or digestive system). Thus, a clinical model may be structured in a hierarchy, thereby expressing refinements of models according to clinical semantics (e.g. a heart valve model might be a refinement of a heart model). Such a clinical model may be represented in the form of an anatomical atlas or map representation of a person, thus enabling visualisation of the anatomical features and available clinical data relating an anatomical feature. An example of a simple clinical model might be a clinical condition where the model might contain two attributes: an anatomical location and a severity.

The anatomical locations might be 'binded' or 'bound' to a value set, where the individual elements might be linked to an area in the map. A clinical model may thus contain all elements from the set, and an instance of the model may then contain only a subset of the elements (e.g. a particular location).

In this regard, it is noted that areas in a clinical map need not be restricted to being linked to elements in a value set. They might also be linked to other levels of aggregation such as models itself (e.g. types of abnormalities)

According to proposed concepts, model elements may be dynamically organized according to a clinical map. For example, the elements may be provided in a value set and presented (e.g. displayed) in a drop-down list next to a clinical map. Thus, if a clinical map is changed, the ordering of the elements may be automatically adjusted so as to reflect the change(s) in the clinical map. For instance, if a clinical map is used for a specific subject, the drop-down list may be adjusted automatically according to the subject-specific clinical map. Further, zooming in a clinical map (e.g. traversing a hierarchy in the clinical maps) may also alter the presentation or ordering of the elements presented.

Some proposed embodiments may therefore be thought of as accounting for a clinical map exploration preference when ordering clinical information. Thus, there may be provided a system that facilitates interaction with clinical information and a clinical map.

Illustrative embodiments may be utilized in many different types of clinical, medical or subject-related environments, such as a hospital, doctor's office, ward, care home, person's home, etc. In order to provide a context for the description of elements and functionality of the illustrative embodiments, the Figures are provided hereafter as examples of how aspects of the illustrative embodiments may be implemented. It should therefore be appreciated the Figures are only examples and are not intended to assert or imply any limitation with regard to the environments, systems or methods in which aspects or embodiments of the present invention may be implemented.

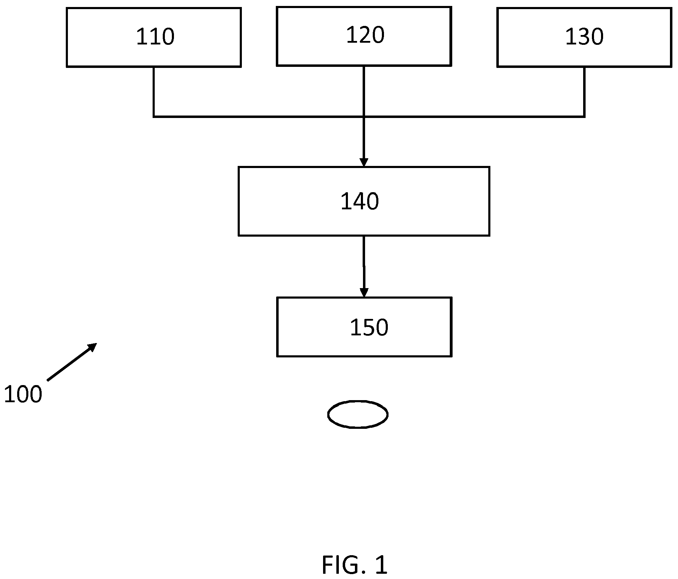

Referring now to Figure 1, there is depicted an exemplary flow diagram of a method 100 for providing ordered clinical information according to an embodiment.

In step 110, a clinical map is obtained. The clinical map comprises graphical information for graphically representing clinical data relating to the subject. For instance, for this exemplary embodiment, the obtained clinical map is representative of an anatomical relationship between anatomical features of a human or animal body. Also, the clinical map comprises hierarchy information representative of a hierarchy of anatomical features of a human or animal body. An example of a clinical map might be a professional 2-D image of coronary segments according to a specific classification system.

In step 120, clinical data relating is obtained. Here, the clinical data comprises a plurality of clinical data elements. For instance, a clinical data element (e.g. obtained from a medical assessment, monitoring instance, medical intervention instance, etc.) includes values of clinical parameters and level information describing a hierarchical level it relates to. An example may also include clinical data regarding an abnormality in a specific segment. An example might be clinical data regarding an abnormality in a specific segment.

In step 130, display preference information representative of a preference for exploring clinical data is obtained. By way of example, the step 130 of obtaining display preference information in this embodiment comprises receiving a user input signal via a user interface, wherein the user input signal comprises display preference information indicating a user's preferred direction for exploring clinical data. In particular, and by way of example only, the display preference information comprises an indication of direction in a graphical representation of the clinical data defined by the clinical map. This may be the direction of an arrow-shaped graphical element, but it will be understood that many other indications of direction may be employed, such as written or spoken indication, a gesture or movement, and/or a visual indication for example. A preference may, for example, comprise the direction of the flow of blood indicated in an image. However, a clinical user in case of multiple vessels might additionally indicate the order of vessels (e.g. first the right coronary artery and second the left coronary artery).

Based on the clinical map and the obtained display preference information, the clinical data is processed (e.g. re-ordered or rearranged) so as to generate an ordered set of clinical data elements in step 140. Here, the processing comprises firstly ordering the plurality of clinical data elements according to the preferred direction for exploring clinical data, thereby generating an ordered list of clinical data elements. Clinical data elements of the ordered list are then sorted based on the clinical map so as to generate a sorted ordered list of clinical data elements.

Also, because, in this example, the clinical data elements comprise level information describing a hierarchical level it relates to, the step of 140 processing the clinical data also includes ordering the plurality of clinical data elements in a hierarchical manner based on the level information. Where the clinical map comprises hierarchy information, the step 140 of processing the clinical data also comprises ordering the plurality of clinical data elements in a hierarchical manner based on the hierarchy information of the clinical map.

Furthermore, as depicted in Figure 1, the proposed embodiment also includes the step 150 of annotating the generated subject-specific clinical model. For instance, one or more graphical representations may be overlaid on a clinical map in a graphical representation of the generated model. This can facilitate quick, easy and intuitive navigation of the clinical model.

It will be appreciated that execution of the method of Figure 1 provides ordered clinical information, and this may then be leveraged for the purpose of displaying information to user (such as a medical professional for example) in a clinical context. Proposed concepts may therefore provide a method for displaying clinical information which incorporates an embodiment of providing ordered clinical information.

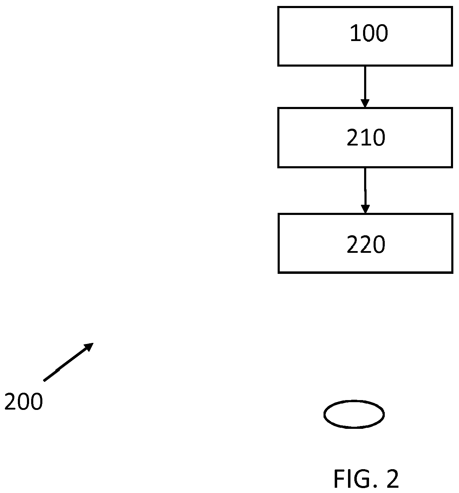

By way of example, Figure 2 depicts an exemplary flow diagram of a method 200 for displaying clinical information according to an embodiment, wherein the method 200 employs the method 100 of Figure 1.

More specifically, the method 200 begins with the step 100 of providing ordered clinical information according to proposed embodiment of Figure 1. Execution of step 100 therefore results in the provision of a clinical information that comprises data elements which are ordered in consideration of a preferred direction for exploring clinical data and a clinical map.

In step 210, a display control signal for displaying clinical information is generated based on the ordered clinical information. The display control signal is for controlling a display device to display the ordered clinical information and may therefore also be generated in consideration of display constraints (such as a display position of the clinical information). In this way, display considerations (such available display space, already-displayed graphical elements, overlapping content, etc.) may be accounted for so as to ensure correct or appropriate display instructions are provided by the control signal.

The clinical data relating to the subject is then displayed in step 220 in accordance with the generated control signal.

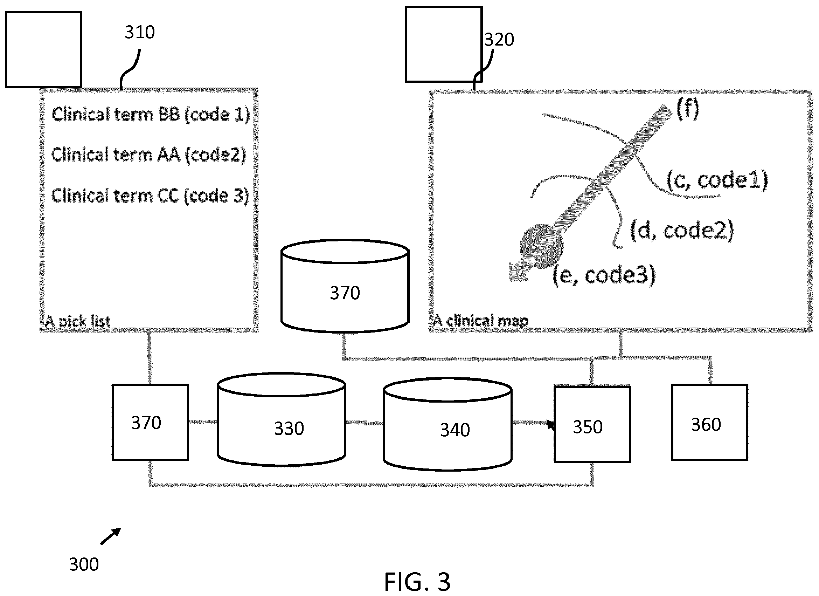

Referring now to Figure 3, there is depicted an exemplary system according to a proposed embodiment. The system 300 is adapted to obtain a 'pick list' display device 310 for displaying a plurality of clinical terms (namely 'Clinical term BB', 'Clinical term AA' and 'Clinical term CC'). The system 300 is also adapted to obtain a 'clinical map' 320 comprising a graphical representation formed from a plurality of graphical elements (c, d, e), wherein each graphical elements is representative of an anatomical feature for example. The graphical elements are codified (e.g. using a coding system such as SNOMED). Furthermore, an indication (which in this example is an arrow f) of a meaningful order of graphical elements is depicted in the clinical map. Here, the arrow f is in a direction that is indicative of a direction from a proximal region to a distal region.

The system 300 is provided with a clinical term database 330 adapted to stored clinical terms that are codified with a code from a coding database 340.

The system 300 also comprises a clinical map coding unit 350 which is configured to associate each graphical element of the clinical map 320 with a code using codes from the coding database 340. By way of example, in the depicted embodiment of Figure 3, the graphical element 'c' is coded by 'code1'.

A clinical flow unit 360 is configured to enable a user to configure the clinical flow (i.e. direction of exploration represented by the arrow labelled 'f') of graphical elements (e.g. anatomical features) in the clinical map 320 by manipulating the direction of the arrow. The flow (e.g. direction of the arrow f) is stored in a clinical flow database 370.

The clinical flow unit 360 is further configured to order graphical elements according to the indicated (anatomical) flow direction (e.g. as indicated by the direction of the arrow f and stored in the clinical flow database 370). Furthermore, the clinical flow unit 360 is arranged to compose a list of sorted codes according to the associated graphical elements (e.g. code1, code2, code3).

The system 300 further comprises a pick list sorting unit 370 that is adapted to sort values in the pick list 310 from the database of terms 330 according to the sorting of codes by the clinical map sorting unit 360.

The system 300 is therefore adapted to order a list according to the graphical elements in the clinical map and a direction of exploration as indicated by the arrow f (e.g. as manipulated by a user). As a result, the pick list 310 can be sorted according to a clinical map and preferences of a specific user.

Also, the clinical flow database 370 may be arranged store user-specific preferences (e.g. clinical patterns per user), thus enabling customisation of ordering to specific users.

In the case of multiple clinical maps, the system 300 may be configured to use a clinical map based on user preference or proximity to a reference map or feature.

Considering now an example in which a heart is displayed, the vessels supplying the heart are in a thin layer at the outside according to specific anatomy. Rotating the heart will not change the anatomy, but the location and order of vessel segments. Embodiments may thus adapt the display of information according to the presentation and user preference(s) (i.e. exploration preference(s). In this way, a user controls may control user interface components to, for example, enter properties of a certain anatomical element, and then graphical elements may be automatically repositioned on a subject-specific clinical image.

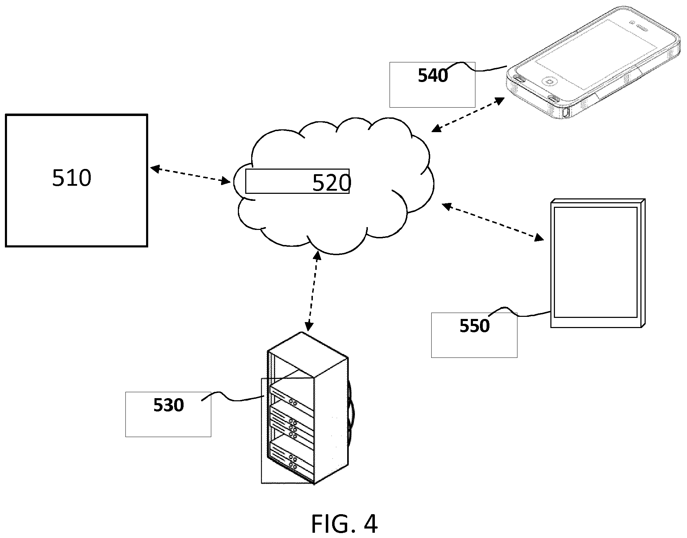

Referring now to Fig. 4, there is depicted an embodiment of a system according to an embodiment of the invention comprising an input system 510 arranged to obtain various sets of information. Here, the input system 510 is adapted to obtain a clinical map, the clinical map comprising graphical information for graphically representing clinical data, to obtain clinical data comprising a plurality of clinical data elements, and to obtain display preference information representative of a preferred direction for exploring clinical data. The input system 510 is adapted to output one or more signals which are representative of obtained information/data.

The input system 510 communicates the output signals via the internet 520 (using a wired or wireless connection for example) to a remotely located data processing system 530 (such as server).

The data processing system 530 is adapted to receive the one or more output signals from the input system 510 and process the received signal(s) to generate an ordered set of clinical data elements based on the clinical map and the obtained display preference information. Thus, the data processing 530 provides a centrally accessible processing resource that can receive information from the input system 510 and run one or more algorithms to order the received information into a specific user-preferred order. Information relating to the ordered clinical data can be stored by the data processing system (for example, in a database) and provided to other components of the system. Such provision of ordered clinical data may be undertaken in response to a receiving a request (via the internet 520 for example) and/or may be undertaken without request (i.e. 'pushed').

For the purpose of receiving information about clinical data from the data processing system 530, and thus to enable ordered clinical data to be viewed, the system further comprises first 540 and second 550 mobile computing devices.

Here, the first mobile computing device 540 is a mobile telephone device (such as a smartphone) with a display for displaying clinical maps and/or clinical data in accordance with embodiments of the proposed concepts. The second mobile computing device 550 is a mobile computer such as a Laptop or Tablet computer with a display for displaying clinical maps and/or clinical data in accordance with embodiments of the proposed concepts.

The data processing system 530 is adapted to communicate clinical model output signals to the first 540 and second 550 mobile computing devices via the internet 520 (using a wired or wireless connection for example). As mentioned above, this may be undertaken in response to receiving a request from the first 540 or second 550 mobile computing devices.

Based on the received output signals, the first 540 and second 550 mobile computing devices are adapted to display one or more graphical elements in a display area provided by their respective display. For this purpose, the first 540 and second 550 mobile computing devices each comprise a software application for processing, decrypting and/or interpreting received clinical data output signals in order to determine how to display graphical elements. Thus, the first 540 and second 550 mobile computing devices each comprise a processing arrangement adapted to determine an attribute of a clinical map or ordered clinical data, and to generate a display control signal for modifying at least one of the size, shape, position, orientation, pulsation or colour of a graphical element based on the determined attribute of the clinical map and/or ordered clinical data.

The system can therefore communicate information about clinical maps and/or ordered clinical data to users of the first 540 and second 550 mobile computing devices. For example, each of the first 540 and second 550 mobile computing devices may be used to display graphical elements to a medical practitioner, doctor, consultant, technician or caregiver for example.

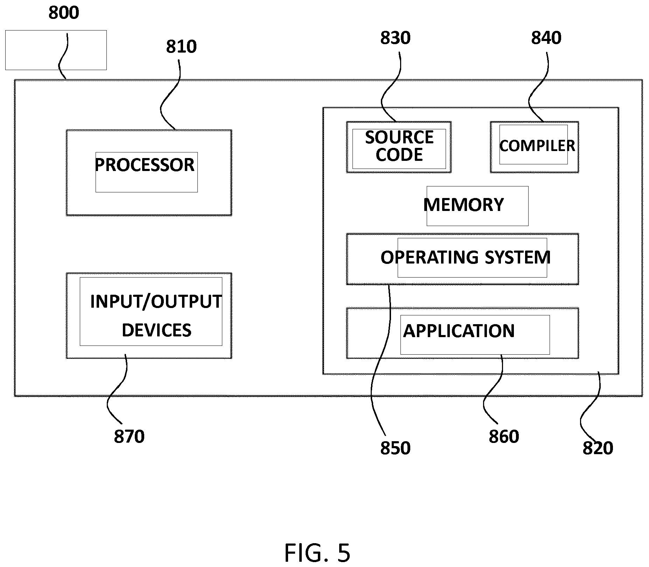

Implementations of the system of Figure 5 may vary between: (i) a situation where the data processing system 530 communicates display-ready clinical data, which may for example comprise display data including graphical elements (e.g. in JPEG or other image formats) that are simply displayed to a user of a mobile computing device using conventional image or webpage display (can be web based browser etc.); to (ii) a situation where the data processing system 530 communicates raw data set information that the receiving mobile computing device then transforms to a clinical map, processes to determine one or more attributes of the clinical map, and then displays graphical elements based on the determined one or more attributes (for example, using local software running on the mobile computing device). Of course, in other implementations, the processing may be shared between the data processing system 530 and a receiving mobile computing device such that part of the clinical model generated at data processing system 530 is sent to the mobile computing device for further processing by local dedicated software of the mobile computing device. Embodiments may therefore employ server-side processing, client-side processing, or any combination thereof.

Further, where the data processing system 530 does not 'push' clinical map information (e.g. clinical model output signals), but rather communicates information in response to receiving a request, the user of a device making such a request may be required to confirm or authenticate their identity and/or security credentials in order for information to be communicated.

Figure 5 illustrates an example of a computer 800 within which one or more parts of an embodiment may be employed. Various operations discussed above may utilize the capabilities of the computer 800. For example, one or more parts of a system for providing subject-specific information (or display unit thereof) may be incorporated in any element, module, application, and/or component discussed herein.

The computer 800 includes, but is not limited to, PCs, workstations, laptops, PDAs, palm devices, servers, storages, and the like. Generally, in terms of hardware architecture, the computer 800 may include one or more processors 810, memory 820, and one or more I/O devices 870 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 840, source code 830, and one or more applications 860 in accordance with exemplary embodiments. As illustrated, the application 860 comprises numerous functional components for implementing the features and operations of the exemplary embodiments. The application 860 of the computer 800 may represent various applications, computational units, logic, functional units, processes, operations, virtual entities, and/or modules in accordance with exemplary embodiments, but the application 860 is not meant to be a limitation.

The operating system 850 controls the execution of other computer programs, and provides scheduling, input-output control, file and data management, memory management, and communication control and related services. It is contemplated by the inventors that the application 860 for implementing exemplary embodiments may be applicable on all commercially available operating systems.

Application 860 may be a source program, executable program (object code), script, or any other entity comprising a set of instructions to be performed. When a source program, then the program is usually translated via a compiler (such as the compiler 840), assembler, interpreter, or the like, which may or may not be included within the memory 820, so as to operate properly in connection with the O/S 850. Furthermore, the application 860 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 870 may include input devices such as, for example but not limited to, a mouse, keyboard, scanner, microphone, camera, etc. Furthermore, the I/O devices 870 may also include output devices, for example but not limited to a printer, display, etc. Finally, the I/O devices 870 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 870 also include components for communicating over various networks, such as the Internet or intranet.

If the computer 800 is a PC, workstation, intelligent device or the like, the software in the memory 820 may further include a basic input output system (BIOS) (omitted for simplicity). The BIOS is a set of essential software routines that initialize and test hardware at startup, start the O/S 850, and support the transfer of data among the hardware devices. The BIOS is stored in some type of read-only-memory, such as ROM, PROM, EPROM, EEPROM or the like, so that the BIOS can be executed when the computer 800 is activated.

When the computer 800 is in operation, the processor 810 is configured to execute software stored within the memory 820, to communicate data to and from the memory 820, and to generally control operations of the computer 800 pursuant to the software. The application 860 and the O/S 850 are read, in whole or in part, by the processor 810, perhaps buffered within the processor 810, and then executed.

When the application 860 is implemented in software it should be noted that the application 860 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 860 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand various embodiments with various modifications are contemplated.

## Claims

1. A method for providing ordered clinical information, the method comprising:
obtaining a clinical map, the clinical map comprising graphical information for graphically representing clinical data;
obtaining clinical data comprising a plurality of clinical data elements;
obtaining display preference information representative of a preference for exploring clinical data; and
processing the clinical data to generate an ordered set of clinical data elements based on the clinical map and the obtained display preference information.

2. The method of claim 1, wherein the clinical data comprises a list of a plurality of clinical data elements, and wherein processing the clinical data comprises:
ordering the plurality of clinical data elements according to the preference for exploring clinical data so as to generate an ordered list of clinical data elements; and
sorting clinical data elements of the ordered list based on the clinical map so as to generate a sorted ordered list of clinical data elements.

3. The method of claim 1 or 2, wherein the clinical map is representative of an anatomical relationship between anatomical features of a human or animal body.

4. The method of any preceding claim, wherein each clinical data element comprises level information describing a hierarchical level it relates to,
and wherein processing the clinical data comprises ordering the plurality of clinical data elements in a hierarchical manner based on the level information of the clinical data elements.

5. The method of any preceding claim, wherein the clinical map comprises hierarchy information representative of a hierarchy of anatomical features of a human or animal body, and wherein processing the clinical data comprises ordering the plurality of clinical data elements in a hierarchical manner based on the hierarchy information of the clinical map.

6. The method of any preceding claim, further comprising:
associating each clinical data element with one or more anatomical features,
and wherein processing the clinical data comprises ordering the plurality of clinical data elements based on the one or more anatomical features associated with each clinical data element and the clinical map.

7. The method of any preceding claim, wherein obtaining display preference information representative of a preference for exploring clinical data comprises:
receiving a user input signal via a user interface, the user input signal comprising display preference information indicating a user's preferred direction or trajectory for exploring clinical data.

8. The method of any preceding claim, wherein the display preference information comprises an indication of direction or trajectory in a graphical representation of the clinical data defined by the clinical map.

9. A method for displaying clinical information, the method comprising:
providing ordered clinical information according to any preceding claim;
generating a display control signal for displaying clinical information based on ordered clinical information; and
displaying clinical information in accordance with the generated display control signal.

10. The method of claim 9, wherein the step of generating a display control signal is further based on a display position of the clinical information.

11. A computer program product for providing or displaying clinical information, wherein the computer program product comprises a computer-readable storage medium having computer-readable program code embodied therewith, the computer-readable program code configured to perform all of the steps of any preceding claim.

12. A computer system comprising: a computer program product according to claim 11; and one or more processors adapted to perform a method according to any of claims 1 to 10 by execution of the computer-readable program code of said computer program product.

13. A system for providing ordered clinical information, the system comprising:
an input interface adapted to obtain a clinical map, the clinical map comprising graphical information for graphically representing clinical data, to obtain clinical data comprising a plurality of clinical data elements, and to obtain display preference information representative of a preference for exploring clinical data; and
a processing module adapted to process the clinical data to generate an ordered set of clinical data elements based on the clinical map and the obtained display preference information.

14. The system of claim 13, wherein the clinical data comprises a list of a plurality of clinical data elements, and wherein the processing module is adapted to:
order the plurality of clinical data elements according to the preference for exploring clinical data so as to generate an ordered list of clinical data elements; and
sort clinical data elements of the ordered list based on the clinical map so as to generate a sorted ordered list of clinical data elements.

15. A system for displaying clinical information, the system comprising:
a system for providing ordered clinical information according to claim 13 or 14;
an output interface adapted to generate a display control signal for displaying clinical information based on ordered clinical information; and
a display unit adapted to display clinical information in accordance with the generated display control signal.
